# EUROPEAN PATENT APPLICATION

(11) **EP 3 970 710 A1**
(43) Date of publication of application: **23.03.2022**
(21) Application number: 20805093.0
(22) Date of filing: 13.05.2020
(51) Int. Cl.: A61K 31/135, A61P 25/16, A61P 25/28, A23L 33/10

(54) **PHARMACEUTICAL COMPOSITION FOR PREVENTING OR TREATING DEGENERATIVE BRAIN DISEASES VIA AUTOPHAGY ACTIVATION**

(30) Priority: 13.05.2019 KR 20190055857
(71) Applicant: VASTHERA Co. Ltd., Seoul 03760 (KR)
(72) Inventor: KWON, Ho Jeong, Seoul 06502 (KR); HWANG, Hui-Yun, Ganghwa-gun Incheon 23040 (KR)
(74) Representative: Ström & Gulliksson AB
(86) International application number: PCT/KR2020/006306
(87) International publication number: WO 2020/231185

(57) **Abstract**

The present invention relates to a pharmaceutical composition for preventing or treating a degenerative brain disease, and a pharmaceutical composition may be provided for preventing or treating a degenerative brain disease, which comprises one or more selected from the group consisting of a compound represented by the following Chemical Formula 1 and a pharmaceutically acceptable salt thereof as an active ingredient.

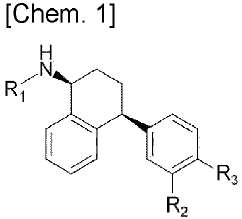

## Description

### [Technical Field]

The present invention relates to a pharmaceutical composition for degenerative brain diseases, more specifically to a pharmaceutical composition for preventing or treating a degenerative brain disease using autophagy activation.

### [Background Art]

As a self-digestion system in which cellular components are degraded and recycled as nutrients and energy sources, autophagy targets aged or dysfunctional organelles and damaged or misfolded proteins, and it modulates proteolytic processes essential for maintaining cellular homeostasis and genetic stability. Autophagy also modulates chemical metabolism and mitigates the production of reactive oxygen species (ROS) by regenerating damaged or longlived mitochondria, and plays a central role in maintaining cellular homeostasis by protecting cells from nutrient starvation, oxygen depletion, invasion of pathogens including bacteria and viruses, and intracellular and extracellular stimuli such as exposure to UV. As a result, when autophagy is not properly modulated, various diseases such as restenosis, neurodegenerative diseases, leukemia, cancer, and aging may occur.

In particular, when the misfolded protein aggregates are accumulated in the cytoplasm, these become cytotoxic substances and thus must be degraded by autophagy. If autophagy is decreased, accumulation of misfolded proteins may be caused, which may lead to neurodegenerative diseases. This system maintains cell homeostasis and causes cell survival or death, but the specific mechanism of when autophagy is induced has not been fully elucidated.

Studies on activating autophagy for the treatment of degenerative brain diseases have been actively conducted in recent years. In general, the modulator that inhibits autophagy is mTOR, and the method of activating autophagy using an mTOR inhibitor is most widely used. Specifically, rapamycin is used to remove amyloid beta (Ab) and tau (tau) in an animal model overexpressing APP and at the same time to enhance cognition, and a tau protein is removed from an animal model overexpressing the tau protein and showing Alzheimer's symptoms. However, in addition to the modulation of autophagy, mTOR has a greatly important role in various intracellular pathways such as NFkB and may thus have a broad role in modulating global gene expression within cells in response to stimuli from various external environments. Therefore, there is a limit to the use of these autophagy activators, which are known to target mTOR as a drug target, as therapeutic agents despite their excellent activity in removing misfolded protein aggregates in degenerative brain diseases.

In addition, studies using autophagy activators targeting substances other than mTOR as a drug target are being conducted. Specifically, in a mouse model, the huntingtin aggregates have been successfully removed by activating autophagy using rilmenidine but not via mTOR, and mutant huntingtin, motility, and lifespan in the brain of a mouse model have been improved using trehalose, which is known to activate autophagy not via mTOR. However, substances which activate autophagy not via mTOR or via two or more targets including mTOR also have limitations in their use as therapeutic agents since these targets have various roles in the intracellular signaling system.

### [Disclosure]

### [Technical Problem]

Accordingly, the technical problem to be solved by the present invention is to provide a pharmaceutical composition for preventing or treating a degenerative brain disease, which does not target an mTOR protein.

The technical problem to be solved by the present invention is also to provide a pharmaceutical composition, which selectively induces autophagy associated with a degenerative brain disease without modulating the expression of another gene and removes a misfolded protein aggregate.

### [Technical Solution]

A pharmaceutical composition according to an embodiment of the present invention for solving the problem described above may be a pharmaceutical composition for preventing or treating a degenerative brain disease, which comprises one or more selected from the group consisting of a compound represented by the following Chemical Formula 1 and a pharmaceutically acceptable salt thereof as an active ingredient.

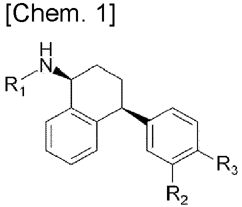

In Chemical Formula 1, R₁ is C₁-C₃ alkyl, R₂ and R₃ are each independently hydrogen or a halogen, and R₂ and R₃ are not hydrogen at the same time.

As used herein, the term "alkyl" refers to a linear or branched saturated hydrocarbon group, and includes, for example, methyl, ethyl, propyl, and isopropyl. C₁-C₃ alkyl refers to an alkyl group having an alkyl unit having 1 to 3 carbon atoms, and the number of carbon atoms in the substituent is not included when the C₁-C₃ alkyl is substituted.

As used herein, the term "halogen" refers to a halogen element, and includes, for example, fluoro, chloro (chlorine), bromo, and iodo.

According to a specific embodiment of the present invention, in Chemical Formula 1, R₁ is C₁ alkyl and R₂ and R₃ are chlorine.

The degenerative brain disease may be selected from the group consisting of Huntington's disease (HD), Parkinson's disease (PD), Alzheimer's disease (AD), prion disease/human mad cow disease, amyotrophic lateral sclerosis (ALS), and any combination of these diseases.

In an embodiment, the compound may bind to a mitochondrial voltage-dependent anion channel-1 (VDAC1), and the compound may bind to aspartic acid 12, alanine 17, valine 20, histidine 184, and serine 196 of the voltage-dependent anion channel.

The compound may bind to an ATP binding domain of the voltage-dependent anion channel and inhibits the ATP binding domain of the voltage-dependent anion channel to induce autophagy, and the compound may bind to the voltage-dependent anion channel by hydrogen bonding and hydrophobic interaction with the voltage-dependent anion channel.

In an embodiment, the compound may induce autophagy due to binding to the voltage-dependent anion channel to degrade amyloid, and the compound may activate the expression of AMP-activated protein kinase (AMPK) in a cell due to binding to the voltage-dependent anion channel to induce autophagy and degrade an intracellular tau protein aggregate.

A health functional food for improving a degenerative brain disease according to an embodiment of the present invention for solving another problem described above may comprise a compound represented by the following Chemical Formula 1 as an active ingredient.

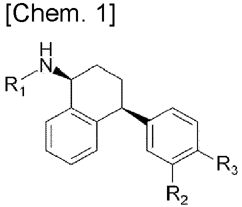

Since the compound that is represented by Chemical Formula 1 and used in the present invention has already been described above, description thereof will be omitted to avoid excessive overlap.

The degenerative brain disease may be a disease selected from the group consisting of Huntington's disease (HD), Parkinson's disease (PD), Alzheimer's disease (AD), prion disease/human mad cow disease, amyotrophic lateral sclerosis (ALS), and any combination of these diseases.

In an embodiment, the compound may bind to a mitochondrial voltage-dependent anion channel-1 (VDAC1), the voltage-dependent anion channel may contain an ATP-binding domain, and the ATP-binding domain may contain aspartic acid 12, alanine 17, valine 20, histidine 184, and serine 196.

The compound may bind to the ATP-binding domain and inhibits the expression of the ATP-binding domain to induce autophagy, and the compound may bind to the voltage-dependent anion channel by hydrogen bonding and hydrophobic interaction with the voltage-dependent anion channel.

The compound may induce autophagy by binding to the voltage-dependent anion channel to degrade amyloid or to degrade an intracellular tau protein aggregate.

A method of degrading a misfolded protein according to an embodiment of the present invention for solving still another problem described above may comprise: binding a compound represented by the following Chemical Formula 1 or a pharmaceutically acceptable salt thereof to a mitochondrial voltage-dependent anion channel by treating an animal cell with the compound or the pharmaceutically acceptable salt thereof to inhibit ATP activity in the cell; inducing activity of adenosine monophosphate-activated protein kinase (AMPK) by the decreased ATP level; inhibiting expression of mTOR and S6K proteins by the activated AMPK; and inducing autophagy in the cell by the inhibited mTOR and S6K activity.

In an embodiment, the compound may bind to an ATP-binding domain of the mitochondrial voltage-dependent anion channel, and the ATP-binding domain may contain aspartic acid 12, alanine 17, valine 20, histidine 184, and serine 196.

According to another aspect of the present invention, the present invention provides a method of preventing or treating a degenerative brain disease, which comprises administering the compound represented by Chemical Formula 1 of the present invention to a subject.

### [Advantageous Effects]

According to an embodiment of the present invention, it is possible to provide a pharmaceutical composition for preventing or treating a degenerative brain disease, the composition comprising sertraline, which binds to voltage-dependent anion channel-1 (VDAC1), decreases ATP levels in the cytoplasm, and activates the expression of AMPK to inhibit mTOR expression, induce autophagy, and degrade tau protein aggregates, and a pharmaceutically acceptable salt thereof.

According to another embodiment of the present invention, it is possible to provide a health functional food for preventing or treating a degenerative brain disease, the food comprising sertraline, which uses voltage-dependent anion channel-1 present in the mitochondrial membrane as a target protein without using mTOR protein as a target protein, unlike conventional autophagy inducers, and thus induces autophagy without directly affecting other major expression *in vivo* such as insulin resistance, which is a disadvantage of inhibitors targeting mTOR protein, and a pharmaceutically acceptable salt thereof.

### [Brief Description of Drawings]

FIGS. 1A and 1B are experimental images on autophagy induction by a pharmaceutical composition for preventing or treating a degenerative brain disease according to an embodiment of the present invention;
FIG. 1C is an image and graph on autophagy induction by a pharmaceutical composition according to an embodiment of the present invention acquired using MDC fluorescence staining;
FIG. 1D is results of an immunoblotting experiment for examining the effect of a pharmaceutical composition according to an embodiment of the present invention on LC3-II and p62;
FIGS. 1E and 1F are images illustrating the activation of autophagic flux by a pharmaceutical composition according to an embodiment of the present invention;
FIG. 2A is results illustrating the effect of a pharmaceutical composition according to an embodiment of the present invention on the cell ATP level in HUVEC measured using an ATPlite luminescence assay system;
FIG. 2B is results of an immunoblotting experiment for examining whether a pharmaceutical composition according to an embodiment of the present invention inhibits mTOR/S6K signaling;
FIG. 2C illustrates the effect of a pharmaceutical composition according to an embodiment of the present invention on EGFP-LC3 puncta;
FIG. 2D illustrates whether autophagy induction by a pharmaceutical composition according to an embodiment of the present invention involves an upstream signaling pathway of mTOR;
FIG. 2E is an image acquired by observing nuclear translocation of TFEB by a pharmaceutical composition according to an embodiment of the present invention;
FIG. 2F is results of Western blotting for examining autophagy induction in a sample treated with a pharmaceutical composition according to an embodiment of the present invention;
FIGS. 3A and 3B illustrate changes in the sensitivity of proteins to hydrolysis caused by binding of a pharmaceutical composition according to an embodiment of the present invention to a small molecule;
FIGS. 3C to 3E are images illustrating the binding sites of a pharmaceutical composition according to an embodiment of the present invention in a protein;
FIGS. 3F and 3G are results of an experiment for determining the starting point of AMPK/mTOR/S6K signaling modulation by a pharmaceutical composition according to an embodiment of the present invention;
FIG. 3H illustrates the activity dependence of a pharmaceutical composition according to an embodiment of the present invention on VDAC1;
FIGS. 4A and 4B illustrate the tauopathy treating effect of a pharmaceutical composition according to an embodiment of the present invention; and
FIG. 5 illustrates a method of inducing autophagy by a pharmaceutical composition according to an embodiment of the present invention.

### [Detailed Description of the Invention]

Hereinafter, preferred embodiments of the present invention will be described in detail with reference to the accompanying drawings.

The embodiments of the present invention are provided to more completely explain the present invention to those of ordinary skill in the art, the following embodiments may be modified in various other forms, and the scope of the present invention is not limited to the following embodiments. Rather, these embodiments are provided so as to fulfill and complete the present invention, and to fully convey the spirit of the present invention to those skilled in the art.

In addition, the thickness or size of each layer in the following drawings is exaggerated for convenience and clarity of description, and the same reference numerals in the drawings refer to the same elements. As used herein, the term "and/or" includes any one and any combination of one or more of those enumerated items.

The terminology used herein is used to describe specific embodiments, but is not to limit the present invention. As used herein, the terms "comprise" and/or "comprising" specify the presence of the recited shapes, numbers, steps, operations, members, elements, and/or groups thereof, but does not exclude the presence or addition of one or more other shapes, numbers, steps, operations, members, elements, and/or groups thereof.

Alzheimer's disease (AD) is a degenerative brain disease, may involve inflammation and defective autophagy, and is characterized by the formation of a pathological plaque complex and an irreversible and massive loss of cognitive abilities due to beta-amyloid and tau aggregates in the neuronal cortex. Therefore, autophagy-inducing compounds may promote the degradation of harmful proteins in the brain of Alzheimer's patients. There is thus a high demand for the development of a potential autophagy-inducing compound that can be used together with other medications for Alzheimer's disease.

In the present specification, in order to provide a pharmaceutical composition for preventing or treating a degenerative brain disease, a phynotypic screening for autophagy induction was performed via the Johns Hopkins Drug Library (JHDL), and antidepressants having autophagy inducing activity, including sertraline and indatraline, were discovered therefrom. Sertraline is a serotonin selective reuptake inhibitor used as an antidepressant after being approved for pharmacological effects with bioactivity in the United States in 1991. In the present specification, among the candidate substances found having autophagy inducing activity, sertraline (trade name: Zoloft), which has excellent activity, has been found to be a pharmaceutical composition for preventing or treating a degenerative brain disease, and the detailed molecular mechanism of the autophagy inducing activity of the pharmaceutical composition and its potential in clinical applications for autophagy-associated diseases will be described.

The pharmaceutical composition for preventing or treating a degenerative brain disease according to an embodiment of the present invention is a compound represented by Chemical Formula 1, where R₁ is C₁ alkyl and R₂ and R₃ are chlorine, namely sertraline (C₁₇H₁₇Cl₂N), which may be represented by the following Chemical Formula 2, and a pharmaceutically acceptable salt thereof. The pharmaceutical composition is a clinical drug having an antidepressant action by inhibition of the serotonin transporter. The pharmaceutical composition can induce conversion of microtubule-associated light chain protein type 3 (LC3-I), which is an important autophagy marker, into LC3-II. LC3 conversion may occur during autophagy induction or at a later step in autophagy inhibition, such as autophagosome-lysosome fusion or lysosome degradation. In addition, the pharmaceutical composition can degrade tau protein and beta amyloid by inducing autophagy. It is thus necessary to recognize the accurate effect of the pharmaceutical composition on autophagic flux.

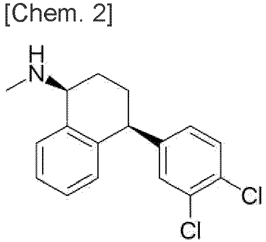

In an embodiment, the pharmaceutical composition comprises one or more selected from the group consisting of sertraline and a pharmaceutically acceptable salt thereof as an active ingredient, and may provide an effect of preventing or treating a degenerative brain disease. The degenerative brain disease may be a disease selected from the group consisting of Huntington's disease (HD), Parkinson's disease (PD), Alzheimer's disease (AD), prion disease/human mad cow disease, amyotrophic lateral sclerosis (ALS), and any combination of these diseases, but is not limited thereto.

The pharmaceutical composition may be prepared in any one formulation selected from the group consisting of tablets, powders, capsules, pills, granules, suspensions, emulsions, syrups, aerosols, external preparations, suppositories, solutions, and injections, but is not limited thereto. In another embodiment, the pharmaceutical composition may be used as an external composition for skin.

In the pharmaceutical composition, sertraline may be used in the form of a pharmaceutically acceptable salt, and as the salt, an acid addition salt formed using a pharmaceutically acceptable free acid may be useful. For example, the acid addition salt may be obtained from inorganic acids such as hydrochloric acid, nitric acid, phosphoric acid, sulfuric acid, hydrobromic acid, hydroiodic acid, nitrous acid, or phosphorous acid and non-toxic organic acids such as aliphatic mono- and dicarboxylates, phenyl-substituted alkanoates, hydroxy alkanoates and alkanedioates, aromatic acids, and aliphatic and aromatic sulfonic acids. The non-toxic salts may include a sulfate, pyrosulfate, bisulfate, sulfite, bisulfite, nitrate, phosphate, monohydrogen phosphate, dihydrogen phosphate, metaphosphate, pyrophosphate chlorite, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycolate, malate, tartrate, methanesulfonate, propanesulfonate, naphthalene-1-sulfonate, naphthalene-2-sulfonate, or mandelate.

The acid addition salt according to the present invention may be prepared by way of a conventional method, for example, by dissolving sertraline in an excess amount of an aqueous acid solution and precipitating the salt using a water-miscible organic solvent, for example, methanol, ethanol, acetone, or acetonitrile. The acid addition salt may also be prepared by heating equal amounts of sertraline and an acid or alcohol in water and evaporating the mixture to dryness or performing suction filtration of the precipitated salt.

In addition, a pharmaceutically acceptable metal salt may be prepared using a base. An alkali metal or alkaline earth metal salt may be obtained, for example, by dissolving the compound in an excess amount of an alkali metal hydroxide or alkaline earth metal hydroxide solution, filtering off the undissolved compound salt, and evaporating the filtrate to dryness. In this case, it is pharmaceutically suitable to prepare a sodium, potassium, or calcium salt as the metal salt. The corresponding silver salt may be obtained by reacting an alkali metal or alkaline earth metal salt with a suitable silver salt (for example, silver nitrate). The pharmaceutical composition of the present invention may include all salts, hydrates, and solvates that may be prepared by conventional methods as well as pharmaceutically acceptable salts.

In an embodiment, the addition salt according to the present invention may be prepared by way of a conventional method. Specifically, the addition salt may be prepared by dissolving sertraline in a water-miscible organic solvent, for example, acetone, methanol, ethanol, or acetonitrile, adding an excessive amount of an organic acid or an aqueous inorganic acid solution, and then performing precipitation or crystallization. Subsequently, the solvent or the excess acid is evaporated from the mixture, and then the residue is dried or suction filtration of the precipitated salt is performed to prepare the addition salt.

When the pharmaceutical composition of the present invention is used as a medicine, the pharmaceutical composition comprising sertraline and/or a pharmaceutically acceptable salt thereof as an active ingredient may be formulated and administered in various oral or parenteral dosage forms at the time of clinical administration, but is not limited thereto. Formulations for oral administration include, for example, tablets, pills, hard/soft capsules, solutions, suspensions, emulsions, syrups, granules, and elixirs, and these formulations contain diluents, for example, lactose, dextrose, sucrose, mannitol, sorbitol, cellulose, and/or glycine and glidants, for example, silica, talc, stearic acid and its magnesium or calcium salts, and/or polyethylene glycol in addition to the active ingredient. Tablets may also contain binders such as magnesium aluminum silicate, starch paste, gelatin, methylcellulose, sodium carboxymethylcellulose, and/or polyvinylpyrrolidine, and may optionally contain disintegrants or effervescent mixtures such as starch, agar, and alginic acid or a sodium salt thereof and/or absorbents, colorants, flavoring agents, and sweeteners.

In another embodiment, the pharmaceutical composition comprising sertraline and/or a pharmaceutically acceptable salt thereof as an active ingredient of the present invention may be administered parenterally. Parenteral administration may be conducted by subcutaneous injection, intravenous injection, intramuscular injection, or intrathoracic injection. In this case, in order to prepare a formulation for parenteral administration, sertraline and/or a pharmaceutically acceptable salt thereof may be mixed in water together with a stabilizer or buffer to prepare a solution or suspension, which may be prepared in an ampoule or vial unit dosage form. The composition may be sterilized and/or contain adjuvants such as preservatives, stabilizers, hydrating agents or emulsifying agents, salts for regulating osmotic pressure, and/or buffers, and other therapeutically useful substances, and may be formulated according to a conventional method such as mixing, granulating, or coating.

The dosage of the pharmaceutical composition of the present invention for the human body may vary depending on the patient's age, weight, sex, administration form, health status, and severity of disease, and is generally 0.001 mg/day to 1,000 mg/day, preferably 0.01 mg/day to 500 mg/day based on an adult patient weighing 60 kg, and the pharmaceutical composition may be administered one time or several times a day at regular time intervals according to the judgment of a doctor or pharmacist.

The present invention provides a quasi-drug composition having an effect of preventing or treating a degenerative brain disease, which comprises one or more selected from the group consisting of sertraline and a pharmaceutically acceptable salt thereof as an active ingredient. When sertraline of the present invention is used as an active ingredient of the quasi-drug composition, sertraline may be added as it is, or may be used together with other quasi-drugs or quasi-drug ingredients, and may be appropriately used according to a conventional method. The active ingredient may be used in a suitable mixed amount depending on the purpose of use.

The quasi-drug composition may be prepared in the form of granules, powders, solutions, creams, ointments, aerosols, pastes, gels, or waxes, and the solution may contain the active ingredient in a state of being dissolved in a solvent, as well as in a state of a suspension or emulsion. Examples of the formulated quasi-drugs include ointments, patches, filter fillers, masks, hand sanitizers, hair products, wet wipes, disinfectants, soaps, or detergent soaps, and may include all quasi-drugs in a conventional sense.

In each formulation, other ingredients may be arbitrarily selected and blended in the quasi-drug composition having an effect of preventing or treating a degenerative brain disease, preferably an amyloid degrading effect according to the formulation or purpose of use of other quasi-drugs. The amount of the active ingredient mixed may be appropriately determined according to the purpose of use, and for example, conventional adjuvants such as thickeners, stabilizers, solubilizers, vitamins, pigments and fragrances, and carriers may be contained. The content of the composition is preferably 0.0001% to 10% by weight based on the total weight, and the stability during the preparation of the composition is low when the content exceeds 10% by weight, and there is a disadvantage in that the effect is insignificant when the content is less than 0.0001% by weight.

The quasi-drug composition comprising sertraline of the present invention as an active ingredient hardly has toxicity and side effects to cells, and may therefore be usefully used as a quasi-drug material.

The present invention provides a cosmetic composition having an effect of preventing or treating a degenerative brain disease, preferably an amyloid degrading effect, which comprises one or more selected from the group consisting of sertraline and a pharmaceutically acceptable salt thereof as an active ingredient. As ingredients contained in the cosmetic composition of the present invention other than sertraline and/or a pharmaceutically acceptable salt thereof as an active ingredient, ingredients commonly used in cosmetic compositions are contained, and for example, conventional adjuvants such as antioxidants, stabilizers, solubilizers, vitamins, pigments and fragrances, and carriers are contained.

The cosmetic composition of the present invention may be prepared in any formulation conventionally prepared in the art, and may be formulated as, for example, solutions, suspensions, emulsions, pastes, gels, creams, lotions, powders, soaps, surfactant-containing cleansing oils, powder foundations, emulsion foundations, wax foundations, and sprays, but is not limited thereto.

The present invention provides a health functional food composition having an effect of preventing or treating a degenerative brain disease, preferably an amyloid degrading effect, which comprises one or more selected from the group consisting of sertraline and a pharmaceutically acceptable salt thereof as an active ingredient. The food composition according to the present invention may be prepared in various forms according to conventional methods known in the art. General food may be prepared by adding sertraline of the present invention to beverages (including alcoholic beverages), fruits and processed foods thereof (for example, canned fruit, canned food, jam, and marmalade), fish, meat and their processed foods (for example, ham and corned beef sausage), breads and noodles (for example, udon, soba, ramen, spaghetti, and macaroni), fruit juice, various drinks, cookies, syrup, dairy products (for example, butter and cheese), edible vegetable oil, margarine, vegetable protein, retort food, frozen food, and various seasonings (for example, doenjang (fermented soybean paste), soy sauce, and sauce), but is not limited thereto. A nutritional supplement may be prepared by adding sertraline of the present invention to a capsule, tablet, pill or the like, but is not limited thereto. Health functional food may be ingested by liquefying, granulating, encapsulating, and powdering sertraline of the present invention itself, for example, in the form of tea, juice, or drink so as to be consumed (health drink), but is not limited thereto. In order to use sertraline of the present invention in the form of a food additive, sertraline may be prepared and used in the form of a powder or a concentrate. The health functional food composition may be prepared in the form of a composition by mixing sertraline of the present invention with an active ingredient known to be effective in preventing or treating a degenerative brain disease.

When sertraline of the present invention is used as a health drink, the health drink composition may contain various flavoring agents or natural carbohydrates as additional ingredients like a conventional drink. The natural carbohydrates may be monosaccharides such as glucose and fructose; disaccharides such as maltose and sucrose; polysaccharides such as dextrin and cyclodextrin; and sugar alcohols such as xylitol, sorbitol, and erythritol. As sweeteners, natural sweeteners such as thaumatin and stevia extract; synthetic sweeteners such as saccharin and aspartame; and the like may be used. The proportion of the natural carbohydrates may be generally about 0.01 g to 0.04 g, preferably about 0.02 g to 0.03 g per 100 mL of the composition of the present invention.

Sertraline of the present invention may be contained as an active ingredient of a health functional food having an anti-stress, anti-depressant, or anti-anxiety effect, and the amount thereof is an amount effective to achieve the amyloid degrading effect, and is not particularly limited, but is preferably 0.01% to 100% by weight based on the total weight of the entire composition. The food composition of the present invention may be prepared by mixing sertraline with other active ingredients known to be effective in preventing or treating a degenerative brain disease. In addition to the above, the health food of the present invention may contain various nutrients, vitamins, electrolytes, flavoring agents, coloring agents, pectic acid, salts of pectic acid, alginic acid, salts of alginic acid, organic acids, protective colloidal thickeners, pH adjusters, stabilizers, preservatives, glycerin, alcohols or carbonating agents, and the like. In addition to the above, the health food of the present invention may contain pulp used in the production of natural fruit juice, fruit juice beverage, or vegetable beverage. These ingredients may be used independently or in mixture. The proportion of these additives is not greatly important, but is generally selected in a range of 0.01 to 0.1 parts by weight per 100 parts by weight of the composition of the present invention.

Hereinafter, the present invention will be described in more detail with reference to Examples. The objects, features, and advantages of the present invention will be easily understood through the following Examples. The present invention is not limited to Examples described herein, and may be embodied in other forms. Examples introduced herein are provided so that the spirit of the present invention can be sufficiently conveyed to those of ordinary skill in the art to which the present invention pertains. Therefore, the present invention should not be limited by the following Examples.

First, in order to demonstrate the autophagy induction by the pharmaceutical composition for preventing or treating a degenerative brain disease according to an embodiment of the present invention and the pharmacological effect for preventing or treating a degenerative brain disease resulting therefrom, an experiment was performed as follows. The pharmaceutical composition of the present invention, sertraline, was screened from 2,386 compounds obtained from the Johns Hopkins Drug Library (JHDL).

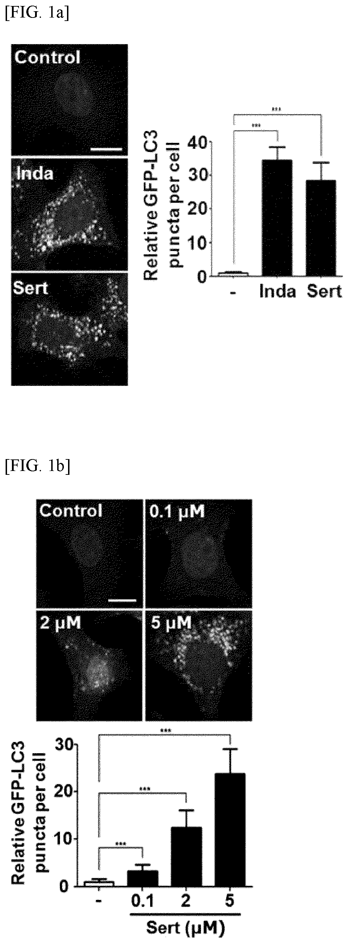

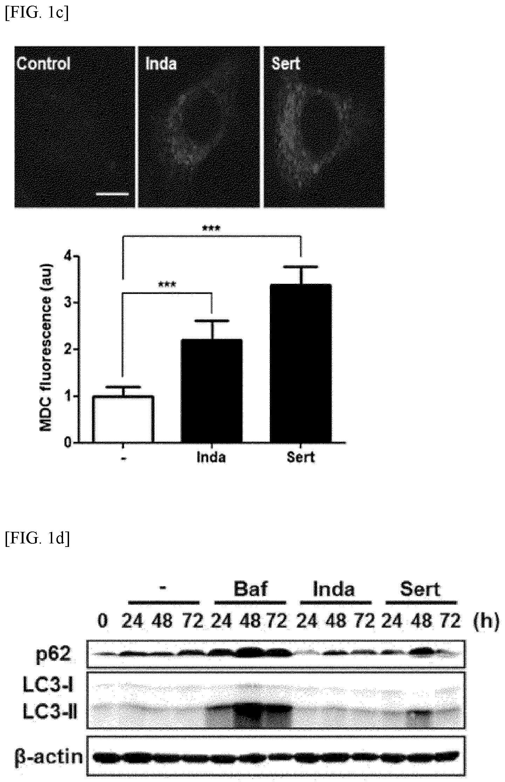

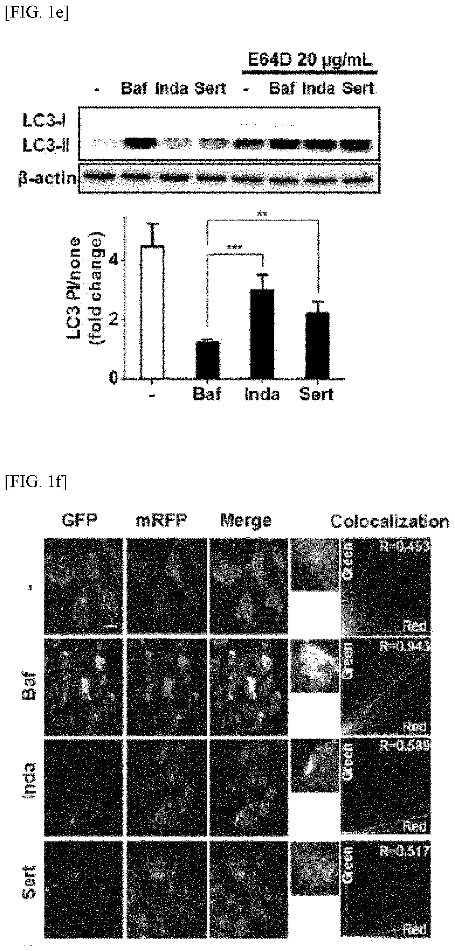

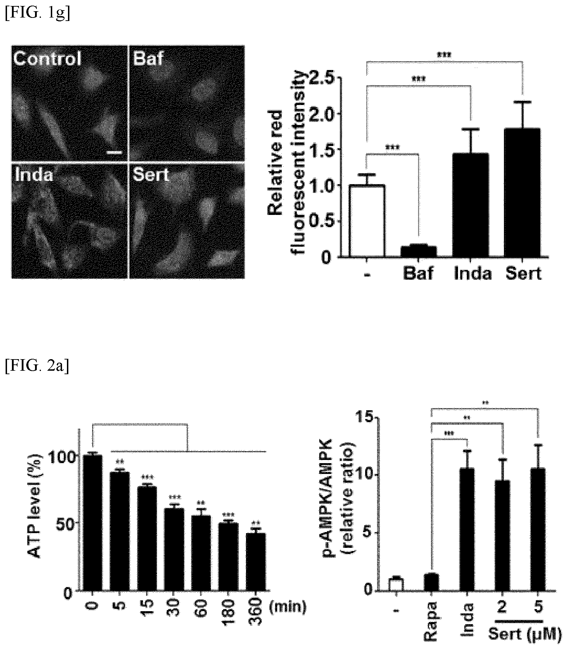

FIGS. 1A to 1G are experimental images on autophagy induction by sertraline, which is the pharmaceutical composition for preventing or treating a degenerative brain disease according to an embodiment of the present invention. FIGS. 1A and 1B illustrate autophagy induction by the pharmaceutical composition according to an embodiment of the present invention observed by immunofluorescence staining, and FIG. 1C is an image and graph on autophagy induction of autophagic vacuoles by the pharmaceutical composition according to an embodiment of the present invention acquired using MDC fluorescence staining. FIG. 1D is results of an immunoblotting experiment for examining the effect of the pharmaceutical composition according to an embodiment of the present invention on LC3-II and p62. FIG. 1E is for examining the degree of autophagy induction by the pharmaceutical composition according to an embodiment of the present invention depending on the presence or absence of E64D, and FIG. 1F is images acquired by observing samples treated with the pharmaceutical composition according to an embodiment of the present invention observed using GFP and/or mRFP fluorescence methods. FIG. 1G is the results acquired by observing the lysosomal activity of the pharmaceutical composition according to an embodiment of the present invention using acridine orange staining.

In an Example, the induction of autophagy by sertraline may be demonstrated by LC3 immunofluorescence staining. Referring to FIG. 1A, as a result of performing immunofluorescence staining of LC3 on a control group (Control) in which the sample is not treated, an experimental group (Inda) in which the sample is treated with indatraline, and an experimental group (Sert) in which the sample is treated with sertraline, which is the pharmaceutical composition of the present invention, it can be seen that the cells treated with sertraline also induce LC3 in the cytoplasm similar to the case of being treated with indatraline, which is known as an antidepressant and induces autophagy from a non-selective monoamine transporter inhibitor. Referring to FIG. 1B, as a result of measuring the induced amount of LC3 in the cytoplasm for samples that are each administered with 0.1 µM, 2 µM, or 5 µM sertraline or a sample that is not administered with sertraline as a control group, it can be seen that LC3 is induced in the cytoplasm in proportion to the amount of sertraline administered.

In an Example, the induction of autophagy by sertraline may be investigated by staining the autophagic vacuoles with monodansylcadaverine (MDC) of a fluorescent dye that binds to autophagic vacuoles. Referring to FIG. 1C, as a result of observing the LC3 expression in a control group (Control) in which autophagic vacuoles are not treated with any pharmaceutical composition, cells (Inda) treated with indatraline, and cells (Sert) treated with sertraline, it can be seen that both the cells treated with indatraline and the cells treated with sertraline express LC3. As a result of performing MDC staining on each of the cells treated with indatraline and the cells treated with sertraline, it has been confirmed that the quantity of LC3 expressed in the cells treated with sertraline is about 1.5 times that in the cells treated with indatraline. In other words, it can be seen that the effect of sertraline on autophagy induction of autophagic vacuoles is significantly superior to that of indatraline.

In order to investigate whether the pharmaceutical composition triggers protein turnover and motility of autophagy, various methods available were used to detect autophagic degradation and to monitor autophagic flux. First, LC3-II and p62 levels were measured by a basic time-dependent immunoblotting method as a method of examining how many autophagy substrates were degraded in a lysosome-dependent manner. Since LC3-II and p62 are only selectively degraded during autophagy, the degradation thereof is widely used to evaluate autophagic flux.

Referring to FIG. 1D, in the case (Sert) of being treated with sertraline, which is the pharmaceutical composition according to an embodiment of the present invention, it can be seen that the levels of LC3-II and p62 increase rapidly for 24 hours, peak at the 48th hour, and then decrease from the 72th hour. This indicates that degradation of LC3-II and p62 proteins occurs at a later stage of autophagy. On the other hand, in the case (Baf) of bafilomycin A of a V-ATPase inhibitor, it is observed that the levels of LC3-II and p62 increase within 24 hours and the high levels of the two types of proteins are maintained during the posttreatment time of 72 hours. This is because the bafilomycin A inhibits autophagic flux. It has been thus confirmed that the pharmaceutical composition of the present invention induces autophagy.

In another Example, the effect of sertraline that was the pharmaceutical composition on the LC3-II levels in the presence and absence of a lysosomal protease inhibitor E64D was investigated. If the treatment with the pharmaceutical composition results in a normal flux state of LC3-II, the expression of LC3-II may further increase in the case of being treated with the pharmaceutical composition and the lysosomal protease inhibitor together than in the case of being treated with only the protease inhibitor.

Referring to FIG. 1E, when the case (Sert on the right in FIG. 1E) of being treated with E64D and the pharmaceutical composition according to an embodiment of the present invention together is compared to the case (Sert on the left in FIG. 1E) of being treated with only the pharmaceutical composition, the LC3-II level further increases in the case of being treated with the pharmaceutical composition and E64D together, and this indicate the activation of autophagic flux by the pharmaceutical composition. On the other hand, in the case (Baf) of bafilomycin A, the expression level of LC3-II is similar regardless of the presence or absence of E64D. Consequently, it can be seen that sertraline, which is the pharmaceutical composition of the present invention, further increases the activity of autophagic flux in the case of being treated with sertraline and a protease inhibitor together.

In order to visualize the change from neutral autophagosomes to acidic autophagosomes based on the different pH stabilities between mRFP-LC3 and GFP-LC3, LC3 double tagged with mRFP/mCherry-GFP may be used. The fluorescence of mRFP is relatively stable in rososomes, but the fluorescence of GFP is unstable in acidic substances. Hence, the autophagic flux may be confirmed by observing that green and red fluorescence by autophagosomes decrease in a local area and red fluorescence by autophagosomes increases. Referring to FIG. 1F, samples treated with bafilomycin A, indatraline, or sertraline are treated with GFP, mRFP, or both GFP and mRFP, and red fluorescence (R value = 0.517) increases when the samples are treated with sertraline, which is the pharmaceutical composition according to an embodiment of the present invention, but yellow fluorescence (R value = 0.943) may be accumulated in HUVECs when the samples are treated with bafilomycin A. Hence, it can be seen that sertraline, which is the pharmaceutical composition of the present invention, significantly induces autophagy.

In an Example, lysosomal activity was investigated using acridine orange staining, which is an analytical method for investigating the role and reliability of lysosomes. Referring to FIG. 1G, treatment with sertraline significantly increases the density of acridine orange, and this indicates that sertraline induces autophagic flux by activating lysosomal activity. Through these results, it has been verified that the pharmaceutical composition according to an embodiment of the present invention activates autophagic flux.

In order to characterize the signaling pathway involved in autophagy induction by the pharmaceutical composition, the effects on the canonical pathway were investigated. The AMPK-mTOR canonical pathway may be activated by generating insufficient cellular energy, and autophagy is induced. FIG. 2A is the results illustrating the effect of the pharmaceutical composition according to an embodiment of the present invention on the cell ATP level in HUVEC measured using the ATPlite luminescence assay system.

Referring to FIG. 2A, in the case of being treated with the pharmaceutical composition for 0 to 360 minutes, it can be seen that the intracellular ATP level decreases in proportion to the time. The increase in the intracellular AMP-ATP ratio activates the AMPK pathway in turn. As a result of measuring the ratio of p-AMPK/AMPK in samples treated with rapamycin, indatraline, or sertraline, it can be seen that relatively high p-AMPK/AMPK is observed in the samples treated with indatraline or sertraline compared to the samples treated with rapamycin, and this indicates that the AMPK pathway is activated.

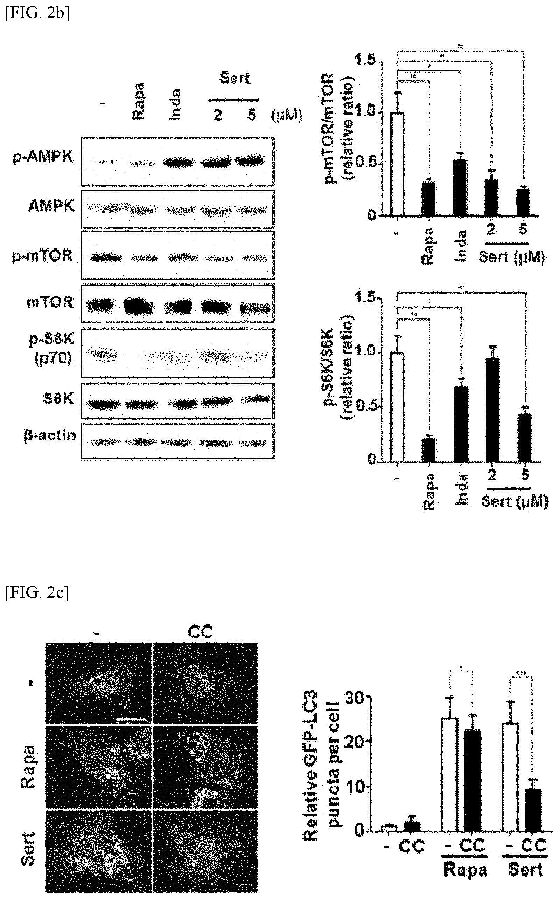

FIG. 2B is the results of an immunoblotting experiment for examining whether the pharmaceutical composition according to an embodiment of the present invention inhibits mTOR/S6K signaling. Referring to FIG. 2B, it can be seen that the pharmaceutical composition inhibits signaling by inducing AMPK activity and decreasing the phosphorylation level of mTOR and its downstream S6K. Specifically, this may be confirmed through the fact that the relative ratio of p-mTOR/mTOR in the case of being treated with sertraline is lower than that in the case of being treated with rapamycin or indatraline. However, it can be seen that the phosphorylation level of AMPK is not affected, phosphorylation of mTOR is directly inhibited, and thus phosphorylation of S6K is also inhibited in the case of being treated with rapamycin. Consequently, the effect of the pharmaceutical composition is distinguished from that in the case of being treated with rapamycin, and the pharmaceutical composition can induce the amplification of AMPK to inhibit the signaling of mTOR and S6K that are downstream of AMPK.

FIG. 2C is images acquired by observing the effect of sertraline, which is the pharmaceutical composition according to an embodiment of the present invention, on EGFP-LC3 puncta. Referring to FIG. 2C, it can be seen that rapamycin-induced autophagy is not significantly affected by the treatment with Compound C but EGFP-LC3 puncta in the cell matrix remarkably decrease by the treatment with Compound C and sertraline together.

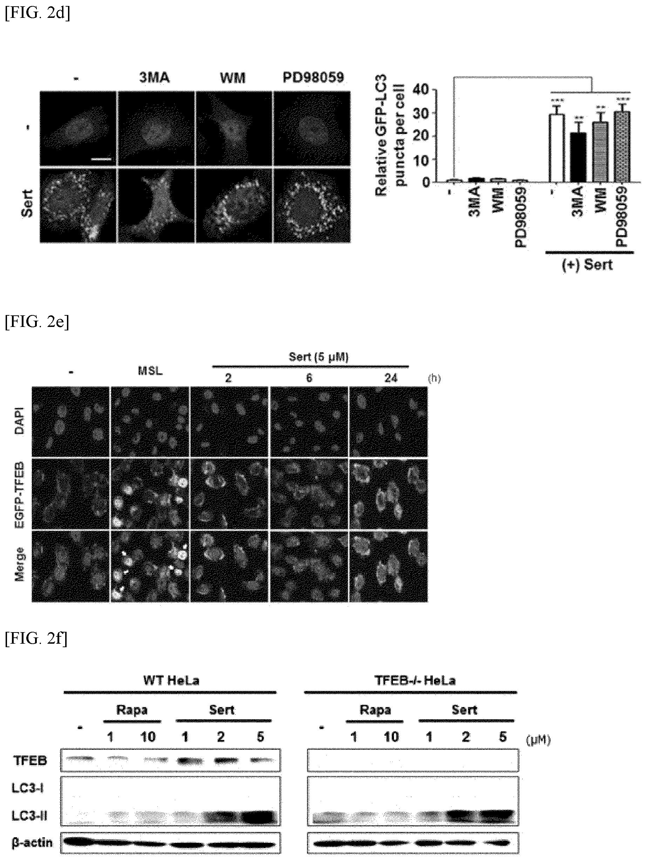

FIG. 2D is an image and a graph as a result of observing that autophagy induction by sertraline, which is the pharmaceutical composition according to an embodiment of the present invention, involves the upstream signaling pathway of mTOR. In order to investigate that autophagy induced by sertraline involved an upstream signaling pathway of mTOR, autophagy inhibitors including 3-MA of a PI3K inhibitor, wortmannin of a PI3K/AKT inhibitor, and PD98059 of a MEK/ERK inhibitor were prepared 1 hour before the treatment with sertraline. Referring to FIG. 2D, it can be seen that the EGFP-LC3-positive puncta remain in the cell matrix despite the presence of each autophagy inhibitor. This result indicates that autophagy induced by sertraline does not modulate the PI3K/AKT and MEK/ERK signaling pathways.

Recently, transcription factor EB (TFEB), a major modulator of autophagy induction and lysosome biogenesis, has been known to communicate with the mTOR signaling pathway. When mTOR is activated and shows its location on the surface of the lysosome via the formation of V-ATPase/Regulator-Rag protein complex, the formed protein complex may phosphorylate TFEB, inhibit nuclear translocation of TFEB, and prevent expression of a target chromosome.

FIG. 2E is an image acquired by directly observing the nuclear translocation of TFEB by infecting HUVECs with the enhanced green fluorescent protein (EGFP)-TFEB plasmid in order to investigate that the pharmaceutical composition according to an embodiment of the present invention operates the translocation of TFEB due to the separation of mTOR from the protein complex. Referring to FIG. 2E, it can be seen that the nuclear translocation of TFEB is not detected during treatment with the pharmaceutical composition for 2 to 24 hours, but MSL, which is known as a small-molecule inducer of TFEB and autophagy, induces the nuclear translocation of TFEB in HUVECs. Consequently, the pharmaceutical composition according to an embodiment of the present invention inhibits phosphorylation of TFEB, enables the nuclear translocation of TFEB, and thus can activate the expression of target chromosome.

FIG. 2F is resulting images of Western blotting for examining autophagy induction in samples treated with the pharmaceutical composition according to an embodiment of the present invention. For the experiment, wild-type Hela cells and Hela cells without TFEB were treated with DMSO, rapamycin, or sertraline of the pharmaceutical composition at the respective concentrations described in FIG. 2F, and Western blotting analysis was performed on the cell extracts to examine autophagy. Referring to FIG. 2F, treatment with the pharmaceutical composition induces conversion to LC3-II in both TFEB+/+ and TFEB-/- HeLa cells. This indicates that the pharmaceutical composition induces autophagy in a TFEB-independent manner.

As mitochondria play an important role in ATP production through oxidative phosphorylation and the pharmaceutical composition activates autophagy independently of the PI3K/AKT and MEK/ERK signaling pathways, voltage-dependent anion channel-1 (hereinafter referred to as VDAC1), which is a channel present in the outer membrane of mitochondria, was selected as a candidate target material of sertraline. It is known that VDAC1 plays an important role in cellular metabolism by transporting ATP and other small metabolites are associated with TOR activity via the outer membrane of mitochondria.

Recently, itraconazole, which is a small-molecule antagonist of VDAC1, has been discovered as a major inhibitor of angiogenesis since itraconazole controls the AMPK/mTOR signaling axis. In order to examine the direct interaction between VDAC1 and sertraline, drug affinity responsive target stability (DARTS) analysis was applied.

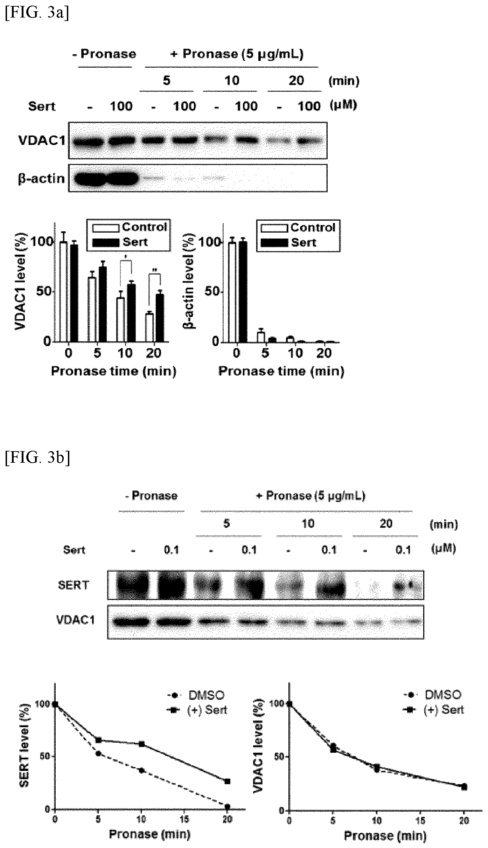

FIG. 3A is the results illustrating changes in the sensitivity of proteins to hydrolysis caused by binding of the pharmaceutical composition according to an embodiment of the present invention to a small molecule. The changes in the sensitivity to hydrolysis are observed using the DARTS method, and the DARTS method is a label-free detection method for target recognition and verification using changes in the sensitivity of proteins to hydrolysis caused by binding to small molecules. Referring to FIG. 3A, it can be seen that VDAC1 exhibits increased stability when VDAC1 and β-actin are treated with sertraline and then pronase is added but β-actin, which is a protein not having binding affinity for sertraline, is degraded by pronase since its sensitivity to proteolysis does not change due to the treatment with sertraline.

FIG. 3B is the results illustrating changes in the sensitivity of proteins to hydrolysis caused by binding of the pharmaceutical composition according to an embodiment of the present invention to another small molecule. In this experiment, DARTS analysis was performed using serotonin reuptake transporter (SRT) protein, which was a known target protein of sertraline associated with antidepressant activity. The desensitization of serotonin reuptake transporter protein induced by sertraline may verify the concentration of nanomolecules. Referring to FIG. 3B, at a nanomolecule concentration, sertraline increases the stability of the pronase of serotonin reuptake transporter protein but not VDAC1 for 20 minutes or more. Hence, the pharmaceutical composition (sertraline) of the present invention may have a greater binding affinity for the serotonin reuptake transport (SRT) protein than for VDAC1.

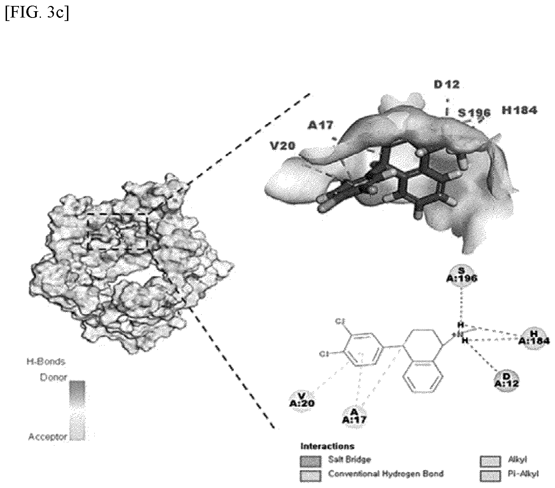

Nevertheless, this may verify that sertraline binds directly to intracellular VDAC1. FIGS. 3C to 3E are images illustrating the binding sites of the pharmaceutical composition according to an embodiment of the present invention in a protein. Referring to FIG. 3C, ATP and DIDS of sertraline may bind to VDAC1 between α-helix and β-sheet in the most stable state, and the binding motif is depicted as high-affinity interaction between sertraline and the VDAC1 pocket. Gray bars on the hydrogen bond surface illustrated denote ligands, orange color denotes hydrophobic interaction, purple color denotes electrostatic interaction, and green and light blue colors denote hydrogen bonding.

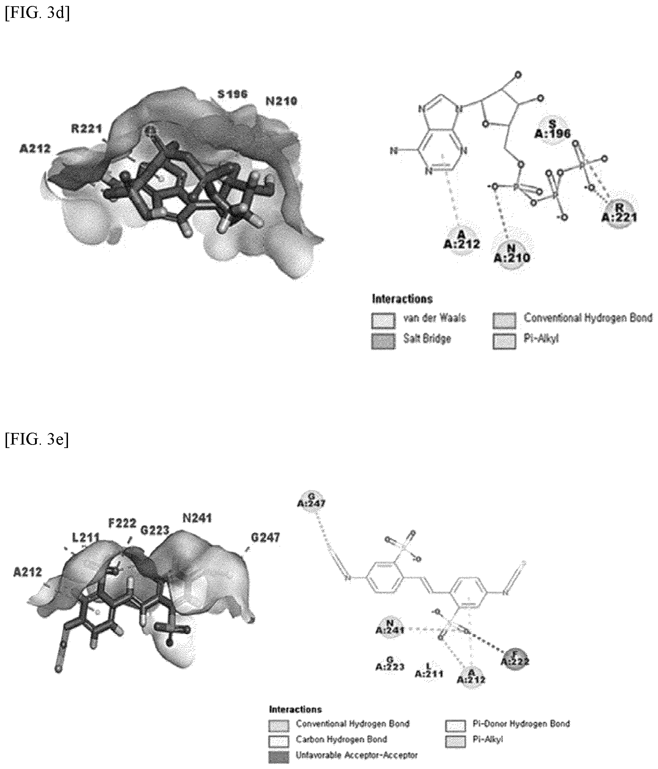

Referring to FIGS. 3D and 3E, it can be seen that the hydrogen bonding (H184, S196) and hydrophobic interaction (A17, V20) between VDAC1 and sertraline impart high bonding strength to VDAC1 as VDAC1 inhibitors ATP(d) and DIDS(e). Preferably, the pharmaceutical composition may bind to the ATP binding domain of VDAC1, which is a voltage-dependent anion channel, and can bind to aspartic acid 12, alanine 17, valine 20, histidine 184, and serine 196 of the ATP binding domain. As such, the pharmaceutical composition bound to the ATP binding domain may induce autophagy in the cell by inhibiting the ATP binding domain.

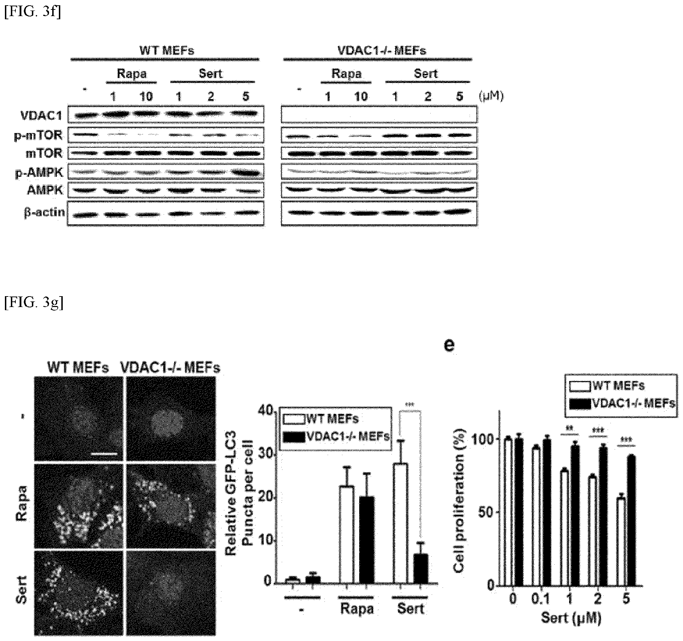

FIGS. 3F and 3G are the results of an experiment for examining whether the modulation of AMPK/mTOR/S6K signaling by the pharmaceutical composition according to an embodiment of the present invention is started by direct binding to VDAC1. In this experiment, VDAC1 wild-type and VDAC1-/- MEFs (mouse embryonic fibroblasts) were used, and wild-type (WT MEFs) and MEFs without VDAC1 (VDAC1-/-MEFs) were both treated with rapamycin (Rapa) and sertraline (Sert). Referring to FIG. 3F, it can be seen that sertraline significantly activates AMPK and inhibits mTOR/S6K phosphorylation in wild-type MEFs. On the other hand, it can be seen that in MEFs without VDAC1, rapamycin still inhibits mTOR activity regardless of VDAC1 expression but sertraline does not modulate AMPK/mTOR/S6K signaling. Referring to FIG. 3G, EGFP-LC3 puncta significantly decrease in MEFs without VDAC compared to wild-type MEFs in the case of being treated with sertraline.

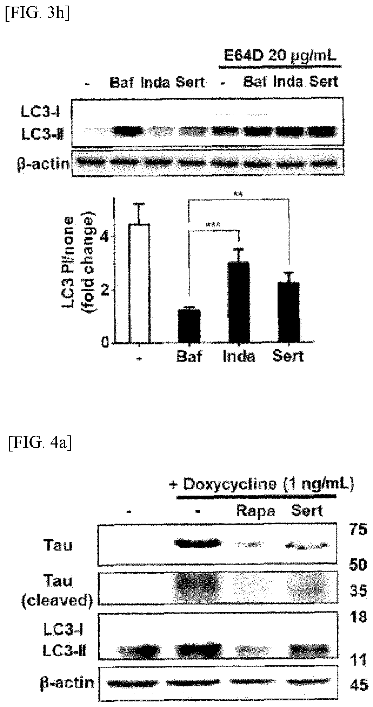

FIG. 3H is the results for illustrating the activity dependence of the pharmaceutical composition according to an embodiment of the present invention on VDAC1. Referring to FIG. 3H, cells were treated with sertraline for 48 hours, and cell proliferation and mitochondrial activity were evaluated. It can be seen that cell proliferation of wild-type MEFs are continuously inhibited by sertraline, but cell proliferation is maintained by 50% or more at the 72nd hour when sertraline is administered to MEFs without VDAC1 in a large amount of about 10 µM. Hence, VDAC1 may be a biologically relevant target protein of sertraline for modulating AMPK/mTOR/S6K signaling and autophagy inducing activity.

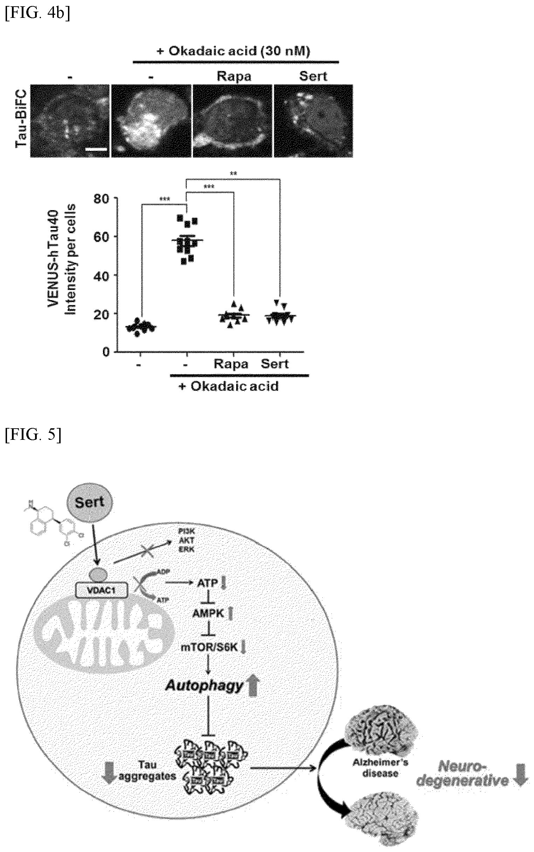

FIGS. 4A and 4B are experimental results on the tauopathy treating effect of the pharmaceutical composition according to an embodiment of the present invention. The pharmaceutical composition according to an embodiment of the present invention may inhibit tauopathy by promoting the degradation of tau protein, and thus may provide an effect of preventing or treating a degenerative brain disease. Tau protein is known to be degraded via autophagy, and used as a substrate since the generation of tau aggregates is particularly important in the early stage of Alzheimer's disease.

Referring to FIG. 4A, it can be seen that both tau and truncated tau decrease in the case of being treated with rapamycin when a sample in which tau and truncated tau are present is treated with rapamycin or sertraline. This effect is because rapamycin attenuates the progression of tauopathy by inducing mTOR-dependent autophagy. Similar to the case of being treated with rapamycin, tau and truncated tau may be significantly decreased in the case of being treated with sertraline as well. In order to visualize and quantify intracellular tau oligomerization, a tau cell line was used in combination with bimolecular fluorescence complementation (BiFC).

Referring to FIG. 4B, the amino-terminal and carboxyl-terminal portions of the Venus protein are independently fused to htau40 and irradiated with a basal fluorescence signal under normal conditions (first lane). However, when chemical induction of tau high-phosphorylation, such as okadaic acid, which is the same as that occurs as a result of tau oligomerization occurs, a strong fluorescence signal may be generated, and thus yellow fluorescent substances are observed in the samples in which tau protein is present (second lane). On the other hand, significantly fewer tau aggregates than those in the cells treated with DMSO are observed in the tau-BiFC cells treated with rapamycin or sertraline (third and fourth lanes). Consequently, sertraline, which is the pharmaceutical composition of the present invention, can promote autophagy to promote degradation of tau protein while proteotoxic stress is caused.

Although phenotype-based screening in clinical drug libraries may be effective, the translation of small molecules for the treatment of autophagy-associated diseases is important to establish the main mechanism of small-molecule activity. Sertraline, which is the pharmaceutical composition according to an embodiment of the present invention, is a selective serotonin transporter inhibitor approved for medicinal use as an antidepressant. It has been elucidated that sertraline is a major substance for inducing autophagy through the present specification. Specifically, a nanomolar concentration level of sertraline is sufficient to bind to the serotonin reuptake transporter in the DARTS analysis, but does not induce autophagy.

FIG. 5 illustrates a method of inducing autophagy by the pharmaceutical composition according to an embodiment of the present invention. In order to elucidate the molecular mechanism by which autophagy induced by sertraline proceeds, a study was conducted on VDAC1 that was a mitochondrial outer membrane protein and a new target protein of sertraline. Referring to FIG. 5 and the above-described Experimental Examples and Examples, sertraline, which is the pharmaceutical composition according to an embodiment of the present invention, can induce autophagy by binding to VDAC1, decreasing intracellular ATP levels, activating AMPK, and inhibiting mTOR. In another Example, sertraline can effectively diminish tau toxic effects by inducing autophagy.

In order to identify target proteins of small molecules without chemical modification, a label-free methodology, DARTS was used. By system target identification including DARTS western analysis, silico docking simulation, and experiments using cells from which VDAC1 has been removed, it can be seen that VDAC1 is a biologically relevant target of sertraline for autophagy-inducing activation.

Pharmaceutical inhibition of VDAC1 by small-molecule inhibitors such as itraconazole and DIDS may indicate a phenotypic association between VDAC1 and mitochondrial metabolism. Inhibition of VDAC1 may prevent Ca²⁺-mediated oxidative stress and apoptosis. The pharmaceutical composition according to an embodiment of the present invention is structurally similar to indatraline, but undergoes a relatively high degree of phosphorylation, and thus may interact with VDAC1 more closely than indatraline. Consequently, the pharmaceutical composition may provide biological activity superior to that of indatraline in autophagy and antiproliferative action in HUVECs and SMCs.

In another Example, VDAC1 has been identified as the main target of the pharmaceutical composition according to an embodiment of the present invention for inducing autophagy, which may be utilized for prevention or treatment of degenerative brain diseases. Autophagy, which modulates VDAC1 by sertraline together with effects independent of apoptosis, may be applied to autophagy therapy without cytotoxicity. The identification of VDAC1 as a target protein of sertraline not only promotes the development of new therapeutic substances for autophagy-associated diseases, but also may provide novel chemical investigations to elucidate the function of VDAC1 in autophagy signaling and autophagy-associated diseases.

The present invention described above is not limited to the above-described embodiments and the accompanying drawings, and it will be apparent to those of ordinary skill in the art to which the present invention pertains that various substitutions, modifications, and changes can be made without departing from the technical spirit of the present invention.

## Claims

1. A pharmaceutical composition for preventing or treating a degenerative brain disease, the composition comprising one or more selected from the group consisting of a compound represented by the following Chemical Formula 1 and a pharmaceutically acceptable salt of the compound as an active ingredient: 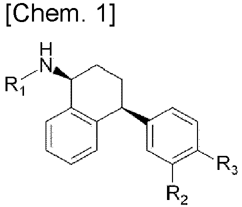 where R₁ is C₁-C₃ alkyl, R₂ and R₃ are each independently hydrogen or a halogen, and R₂ and R₃ are not hydrogen at the same time.

2. The pharmaceutical composition according to claim 1, wherein R₁ is C₁ alkyl and R₂ and R₃ are chlorine in Chemical Formula 1.

3. The pharmaceutical composition according to claim 1, wherein the degenerative brain disease is a disease selected from the group consisting of Huntington's disease (HD), Parkinson's disease (PD), Alzheimer's disease (AD), prion disease/human mad cow disease, amyotrophic lateral sclerosis (ALS), and any combination of these diseases.

4. The pharmaceutical composition according to claim 1, wherein the compound binds to a mitochondrial voltage-dependent anion channel-1 (VDAC1).

5. The pharmaceutical composition according to claim 4, wherein the compound binds to aspartic acid 12, alanine 17, valine 20, histidine 184, and serine 196 of the voltage-dependent anion channel.

6. The pharmaceutical composition according to claim 4, wherein the compound binds to an ATP binding domain of the voltage-dependent anion channel and inhibits the ATP binding domain of the voltage-dependent anion channel to induce autophagy.

7. The pharmaceutical composition according to claim 4, wherein the compound binds to the voltage-dependent anion channel by hydrogen bonding and hydrophobic interaction with the voltage-dependent anion channel.

8. The pharmaceutical composition according to claim 4, wherein the compound induces autophagy due to binding to the voltage-dependent anion channel to degrade amyloid.

9. The pharmaceutical composition according to claim 4, wherein the compound activates expression of AMP-activated protein kinase (AMPK) in a cell due to binding to the voltage-dependent anion channel to induce autophagy and degrade an intracellular tau protein aggregate.

10. A functional food composition for improving a degenerative brain disease, the composition comprising one or more selected from the group consisting of a compound represented by the following Chemical Formula 1 and a pharmaceutically acceptable salt of the compound as an active ingredient: 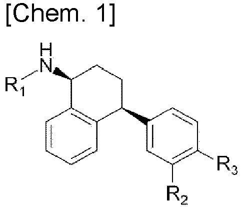 where R₁ is C₁-C₃ alkyl, R₂ and R₃ are each independently hydrogen or a halogen, and R₂ and R₃ are not hydrogen at the same time.

11. The functional food composition according to claim 10, wherein R₁ is C₁ alkyl and R₂ and R₃ are chlorine in Chemical Formula 1.

12. The functional food composition according to claim 10, wherein the degenerative brain disease is a disease selected from the group consisting of Huntington's disease (HD), Parkinson's disease (PD), Alzheimer's disease (AD), prion disease/human mad cow disease, amyotrophic lateral sclerosis (ALS), and any combination of these diseases.

13. The functional food composition according to claim 10, wherein the compound binds to a mitochondrial voltage-dependent anion channel-1 (VDAC1).

14. The functional food composition according to claim 13, wherein
the voltage-dependent anion channel contains an ATP-binding domain, wherein
the ATP-binding domain contains aspartic acid 12, alanine 17, valine 20, histidine 184, and serine 196.

15. The functional food composition according to claim 14, wherein the compound binds to the ATP-binding domain and inhibits expression of the ATP-binding domain to induce autophagy.

16. The functional food composition according to claim 13, wherein the compound binds to the voltage-dependent anion channel by hydrogen bond and hydrophobic interaction with the voltage-dependent anion channel.

17. The functional food composition according to claim 13, wherein the compound induces autophagy by binding to the voltage-dependent anion channel to degrade amyloid.

18. The functional food composition according to claim 13, wherein the compound induces autophagy by binding to the voltage-dependent anion channel to degrade an intracellular tau protein aggregate.

19. A method of degrading a misfolded protein in a subject, the method comprising administering a compound represented by the following Chemical Formula 1 or a pharmaceutically acceptable salt of the compound to the subject: 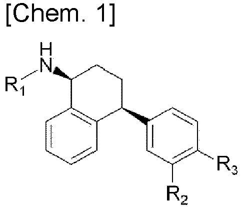 where R₁ is C₁-C₃ alkyl, R₂ and R₃ are each independently hydrogen or a halogen, and R₂ and R₃ are not hydrogen at the same time.

20. The method according to claim 19, wherein R₁ is C₁ alkyl and R₂ and R₃ are chlorine in Chemical Formula 1.
